# EUROPEAN PATENT APPLICATION

(11) **EP 4 810 481 A1**
(43) Date of publication of application: **23.09.2026**
(21) Application number: 24891687.6
(22) Date of filing: 01.11.2024
(51) Int. Cl.: C07D 207/267, B01D 3/14

(54) **PURIFICATION METHOD AND PURIFICATION APPARATUS FOR NMP**

(30) Priority: 13.11.2023 KR 20230156769
(71) Applicant: Duksan Co., Ltd., Anseong-si, Gyeonggi-do 17601 (KR); Hanmo Corporation, Seoul 08390 (KR)
(72) Inventor: LEE, Seungyong, Hongseong-gun, Chungcheongnam-do 32210 (KR); LEE, Wooyong, Yongin-si, Gyeonggi-do 16990 (KR); LEE, Heejae, Bucheon-si, Gyeonggi-do 14581 (KR); YOO, Insung, Seoul 08848 (KR)
(74) Representative: AWA Sweden AB
(86) International application number: PCT/KR2024/016986
(87) International publication number: WO 2025/105740

(57) **Abstract**

The present invention relates to a purification method and purification apparatus for NMP. Specifically, according to one embodiment of the present invention, the purification method for NMP comprises the steps of: (1) distilling waste NMP in a primary distillation column so as to primarily purify same; (2) discharging an effluent containing water, separated out through the primary purification, to the outside of the system through the top of the primary distillation column, and discharging a primary bottom liquid through the bottom of the primary distillation column; (3) injecting the discharged primary bottom liquid into a secondary distillation column and secondarily purifying same; (4) discharging the purified NMP, separated out through the secondary purification, to the outside of the system through the top of the secondary distillation column, and discharging the secondary bottom liquid through the bottom of the secondary distillation column; (5) injecting the discharged secondary bottom liquid into a filtrate recovery device and heat treating same; (6) discharging a tertiary bottom liquid, separated out through the heat treatment, to the outside of the system through the bottom of the filtrate recovery device, and discharging a gas mixture containing NMP through the top of the filtrate recovery device; and (7) injecting the discharged gas mixture into the secondary distillation column, and thus the recovery rate of NMP recovered from waste NMP is improved such that high-purity NMP can be provided.

## Description

### Technical Field

The present invention relates to a process and an apparatus for purifying waste NMP.

### Background Art

In recent years, as technological development for portable devices, such as mobile phones and cameras, has been expedited, interest in secondary batteries as an energy source has increased. While demand for lithium secondary batteries, which have a high energy density and operating potential, a long cycle life, and a low self-discharge rate, is rapidly increasing, among such secondary batteries, research on their recycling is also ongoing.

N-methyl-2-pyrrolidone (NMP), an organic solvent, is widely used as a solvent in industries, such as petrochemicals, plastics, and pharmaceuticals, by virtue of its chemical stability, low volatility, and ability to dissolve various substances. It is also used in the process of manufacturing secondary batteries.

In particular, a lithium ion secondary battery is composed of a positive electrode manufactured by applying an electrode raw material comprising a lithium compound such as lithium cobaltate or lithium manganate, a binder such as polyvinylidene fluoride, and N-methyl-2-pyrrolidone (NMP) as a solvent to a substrate and firing the electrode raw material; and a negative electrode manufactured by applying an electrode raw material comprising a compound comprising carbon, titanium, and the like, a binder such as polyvinylidene fluoride, and water as a solvent to a substrate and firing the electrode raw material. As described above, since NMP is not only used as a main material for manufacturing positive electrodes in the electrode manufacturing process, but is also used in cleaning and drying steps, it is discharged in large quantities. Thus, research is ongoing on technologies to effectively recover it.

For example, Korean Patent No. 10-2050990 discloses a system for separating and recovering NMP from NMP gas in which NMP-containing gas that contains NMP is brought into gas-liquid contact with water that has absorbed NMP, thereby allowing NMP in the NMP-containing gas to be absorbed into the water.

### [Prior Art Document]

### [Patent Document]

(Patent Document 1) Korean Patent No. 10-2050990

### Detailed Description of the Invention

### Technical Problem

Accordingly, the present invention aims to provide a process and an apparatus for purifying NMP, which can enhance the recovery rate of NMP to be recovered from waste NMP and provide high-purity NMP.

### Solution to the Problem

A process for purifying NMP according to an embodiment of the present invention comprises: (1) distilling waste NMP in a first distillation column to perform first purification; (2) discharging an overhead product containing water separated by the first purification to the outside of the system through the top of the first distillation column; and discharging a first bottom product through the bottom of the first distillation column; (3) feeding the discharged first bottom product to a second distillation column to perform second purification; (4) discharging purified NMP separated by the second purification to the outside of the system through the top of the second distillation column; and discharging a second bottom product through the bottom of the second distillation column; (5) feeding the discharged second bottom product to a filtrate recovery device to perform thermal treatment thereof; (6) discharging a third bottom product separated by the thermal treatment to the outside of the system through the bottom of the filtrate recovery device; and discharging a gas mixture containing NMP through the top of the filtrate recovery device; and (7) feeding the discharged gas mixture to the second distillation column.

An apparatus for purifying NMP according to another embodiment of the present invention comprises: a supply unit for supplying waste NMP; a first distillation column for performing first purification of the supplied waste NMP through distillation, discharging an overhead product containing water separated by the first purification to the outside of the system through its top, and discharging a first bottom product through its bottom; a second distillation column for performing second purification of the first bottom product through distillation, discharging purified NMP separated by the second purification to the outside of the system through its top, and discharging a second bottom product through its bottom; and a filtrate recovery device for performing thermal treatment of the second bottom product, discharging a third bottom product separated by the thermal treatment to the outside of the system through its bottom, and discharging a gas mixture containing NMP through its top.

### Advantageous Effects of the Invention

The process for purifying NMP according to an embodiment of the present invention comprises: (1) distilling waste NMP in a first distillation column to perform first purification; (2) discharging an overhead product containing water separated by the first purification to the outside of the system through the top of the first distillation column; and discharging a first bottom product through the bottom of the first distillation column; (3) feeding the discharged first bottom product to a second distillation column to perform second purification; (4) discharging purified NMP separated by the second purification to the outside of the system through the top of the second distillation column; and discharging a second bottom product through the bottom of the second distillation column; (5) feeding the discharged second bottom product to a filtrate recovery device to perform thermal treatment thereof; (6) discharging a third bottom product separated by the thermal treatment to the outside of the system through the bottom of the filtrate recovery device; and discharging a gas mixture containing NMP through the top of the filtrate recovery device; and (7) feeding the discharged gas mixture to the second distillation column. As a result, the recovery rate of NMP to be recovered from waste NMP can be enhanced, and high-purity NMP can be provided.

In particular, water in the waste NMP can be effectively removed through the first purification, and substances with a boiling point higher than that of NMP that may be contained in the third bottom product discharged by the second purification can be effectively removed by thermal treatment in the filtrate recovery device. In addition, as the gas mixture containing NMP separated by thermal treatment in the filtrate recovery device is fed again to the second distillation column, the recovery rate and purity of NMP can be further enhanced.

### Brief Description of the Drawing

Fig. 1 schematically illustrates a flow chart of an NMP purification process according to an embodiment of the present invention.
Fig. 2 illustrates an example of an NMP purification apparatus according to another embodiment of the present invention.

### Best Mode for Carrying out the Invention

Hereinafter, the present invention will be described in detail. The present invention is not limited to the disclosures given below, but it may be modified into various forms as long as the gist of the invention is not changed.

Throughout the present specification, when a part is referred to as "comprising" an element, it is understood that other elements may be comprised, rather than other elements are excluded, unless specifically stated otherwise.

All numbers and expressions related to the quantities of components, reaction conditions, and the like used herein are to be understood as being modified by the term "about," unless otherwise indicated.

In the present specification, in the case where an element is mentioned to be formed "on" or "under" another element, it means not only that one element is directly formed "on" or "under" another element, but also that one element is indirectly formed on or under another element with other element(s) interposed between them.

Throughout the present specification, the terms first, second, and the like are used to describe various components. But the components should not be limited by the terms. The terms are used only for the purpose of distinguishing one component from another.

### NMP purification process

The process for purifying NMP according to an embodiment of the present invention comprises: (1) distilling waste NMP in a first distillation column to perform first purification; (2) discharging an overhead product containing water separated by the first purification to the outside of the system through the top of the first distillation column; and discharging a first bottom product through the bottom of the first distillation column; (3) feeding the discharged first bottom product to a second distillation column to perform second purification; (4) discharging purified NMP separated by the second purification to the outside of the system through the top of the second distillation column; and discharging a second bottom product through the bottom of the second distillation column; (5) feeding the discharged second bottom product to a filtrate recovery device to perform thermal treatment thereof; (6) discharging a third bottom product separated by the thermal treatment to the outside of the system through the bottom of the filtrate recovery device; and discharging a gas mixture containing NMP through the top of the filtrate recovery device; and (7) feeding the discharged gas mixture to the second distillation column.

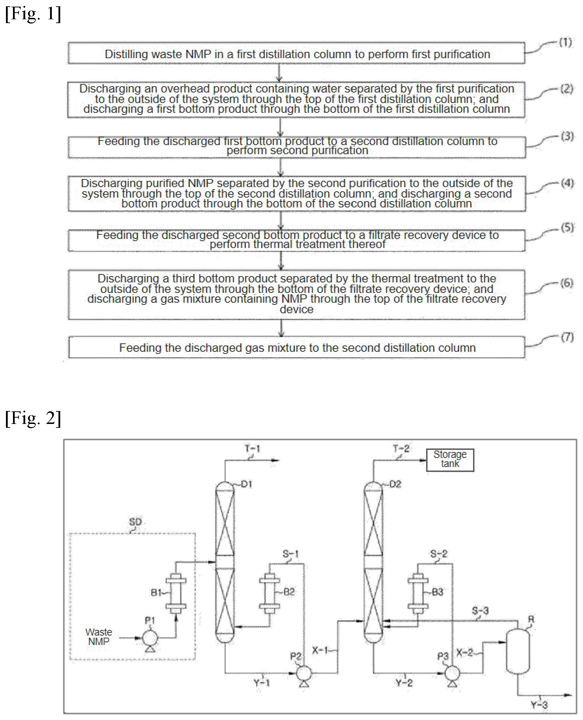

Fig. 1 schematically illustrates a flow chart of an NMP purification process according to an embodiment of the present invention. Fig. 2 illustrates an example of an NMP purification apparatus according to another embodiment of the present invention. Specifically, Fig. 2 illustrates an NMP purification apparatus composed of a supply unit (SD), a first distillation column (D1), a second distillation column (D2), and a filtrate recovery device (R).

Hereinafter, the process for purifying NMP according to an embodiment of the present invention will be described with reference to Figs. 1 and 2.

### Step (1) of distilling waste NMP in a first distillation column to perform first purification

The process for purifying NMP according to an embodiment of the present invention comprises a step of distilling waste NMP in a first distillation column to perform first purification.

The waste NMP may be recovered once it has been used in the process of manufacturing a lithium secondary battery. For example, the waste NMP may be recovered once it has been used as a raw material for a lithium secondary battery, but it is not limited thereto.

The waste NMP may contain NMP in an amount of 70% by weight or more. For example, the content of NMP in the waste NMP may be 72% by weight or more, 75% by weight or more, 78% by weight or more, 80% by weight or more, 85% by weight or more, 90% by weight or more, or 95% by weight or more, based on the total weight of the waste NMP.

In addition, the waste NMP may contain water. For example, the content of water in the waste NMP may be 30% by weight or less, 25% by weight or less, 20% by weight or less, or 15% by weight or less, based on the total weight of the waste NMP.

In step (1), the waste NMP may be fed to the middle or upper part of the first distillation column. Specifically, the waste NMP may preferably be fed to the middle position, or an upper position, of the first distillation column in order to perform the first purification more efficiently, but it is not limited thereto.

The first purification in step (1) may be performed at a temperature of 50°C to 150°C and a pressure of 100 Torr to 130 Torr. For example, the first purification may be performed at a temperature of 52°C to 145°C or 55°C to 143°C and a pressure of 105 Torr to 130 Torr or 110 Torr to 125 Torr. As the temperature and pressure conditions of the first purification satisfy the above ranges, water contained in the waste NMP can be effectively removed.

### Step (2) of discharging an overhead product containing water separated by the first purification to the outside of the system through the top of the first distillation column; and discharging a first bottom product through the bottom of the first distillation column

The process for purifying NMP according to an embodiment of the present invention comprises a step of discharging an overhead product containing water separated by the first purification to the outside of the system through the top of the first distillation column; and discharging a first bottom product through the bottom of the first distillation column.

Specifically, in step (2), the overhead product containing water is separated from the waste NMP in the first purification of step (1) and discharged to the outside of the system, which reduces the content of water, thereby enhancing the purity of waste NMP.

The content of water in the first bottom product may be 0.01% or less. For example, the content of water in the first bottom product may be 0.008% or less, 0.006% or less, or 0.005% or less.

In addition, the first bottom product may contain NMP in an amount of 99.9% by weight or more. For example, the content of NMP in the first bottom product may be 99.92% by weight or more, 99.94% by weight or more, or 99.95% by weight or more, based on the total weight of the first bottom product. As most of the water in the waste NMP is effectively removed in the first purification described above, the purity of NMP in the first bottom product can be further enhanced.

### Step (3) of feeding the discharged first bottom product to a second distillation column to perform second purification

The process for purifying NMP according to an embodiment of the present invention comprises a step of feeding the discharged first bottom product to a second distillation column to perform second purification.

The purity of NMP can be enhanced through the second purification. Specifically, the waste NMP may contain a component with a boiling point higher than that of NMP, along with NMP. As the second purification is performed in step (3) on the first bottom product, from which water has been removed in the first purification, the component having a boiling point higher than that of NMP can be effectively removed, thereby further enhancing the purity of NMP.

In step (3), the first bottom product may be fed to the middle or lower part of the second distillation column. Specifically, the first bottom product may preferably be fed to the middle position, or a lower position, of the second distillation column in order to perform the second purification more efficiently, but it is not limited thereto.

The second purification may be performed at a temperature of 120°C to 150°C and a pressure of 50 Torr to 90 Torr. For example, the second purification may be performed at a temperature of 122°C to 148°C or 126°C to 145°C and a pressure of 55 Torr to 85 Torr or 65 Torr to 80 Torr. As the temperature and pressure conditions of the second purification satisfy the above ranges, the component having a boiling point higher than that of NMP and contained in the first bottom product can be effectively removed.

### Step (4) of discharging purified NMP separated by the second purification to the outside of the system through the top of the second distillation column; and discharging a second bottom product through the bottom of the second distillation column

The process for purifying NMP according to an embodiment of the present invention comprises a step of discharging purified NMP separated by the second purification to the outside of the system through the top of the second distillation column; and discharging a second bottom product through the bottom of the second distillation column.

Specifically, in step (4), as a second bottom product containing the component with a boiling point higher than that of NMP is separated from the first bottom product by the second purification in step (3) and discharged, purified NMP can be obtained.

The second bottom product may contain the component having a boiling point higher than that of NMP in an amount of 1.0% by weight or less. For example, the content of the component having a boiling point higher than that of NMP in the second bottom product may be 0.8% by weight or less, 0.6% by weight or less, or 0.4% by weight or less, based on the total weight of the second bottom product.

The purified NMP may have a purity of 99.9% or higher. For example, the purity of the purified NMP may be 99.92% or more, 99.94% or more, or 99.95% or more.

### Step (5) of feeding the discharged second bottom product to a filtrate recovery device to perform thermal treatment thereof

The process for purifying NMP according to an embodiment of the present invention comprises a step of feeding the discharged second bottom product to a filtrate recovery device to perform thermal treatment thereof.

Even if water and the component with a boiling point higher than that of NMP contained in the waste NMP are removed by the first and second purification steps described above, the second bottom product discharged by the second purification may contain NMP.

The second bottom product may contain NMP in an amount of 99% by weight or more. For example, the content of NMP in the second bottom product may be 99.2% by weight or more, 99.4% by weight or more, or 99.6% by weight or more, based on the total weight of the second bottom product.

As the process for purifying NMP according to an embodiment of the present invention comprises a step of feeding the second bottom product to the filtrate recovery device and performing thermal treatment, NMP contained in the second bottom product can be separated in a gaseous form. As it is fed to the second distillation column to perform second purification, the recovery rate of NMP can be enhanced.

In step (5), the thermal treatment may be performed at a temperature of 110°C to 140°C and a pressure of 60 Torr to 100 Torr under stirring at 20 rpm to 80 rpm. For example, the thermal treatment may be performed at a temperature of 115°C to 140°C, 125°C to 138°C, or 128°C to 135°C, and a pressure of 65 Torr to 95 Torr or 75 Torr to 85 Torr, under stirring at 25 rpm to 70 rpm or 30 rpm to 60 rpm. As the thermal treatment conditions satisfy the above ranges, the recovery rate of NMP can be further enhanced.

### Step (6) of discharging a third bottom product separated by the thermal treatment to the outside of the system through the bottom of the filtrate recovery device; and discharging a gas mixture containing NMP through the top of the filtrate recovery device

The process for purifying NMP according to an embodiment of the present invention comprises a step of discharging a third bottom product separated by the thermal treatment to the outside of the system through the bottom of the filtrate recovery device; and discharging a gas mixture containing NMP through the top of the filtrate recovery device.

Specifically, in step (6), the component having a boiling point higher than that of NMP can be separated from the second bottom product once again as the third bottom product is discharged by the thermal treatment in step (5), and a gas mixture containing NMP can be obtained from the second bottom product.

The third bottom product may contain the component having a boiling point higher than that of NMP in an amount of 1.5% by weight or less. For example, the content of the components having a boiling point higher than that of NMP in the third bottom product may be 1.0% by weight or less, 0.8% by weight or less, or 0.6% by weight or less, based on the total weight of the third bottom product.

In addition, the gas mixture may contain NMP in an amount of 98.5% by weight or more. For example, the content of NMP in the gas mixture may be 99.0% by weight or more, 99.2% by weight or more, or 99.4% by weight or more, based on the total weight of the gas mixture.

### Step (7) of feeding the discharged gas mixture to the second distillation column

The process for purifying NMP according to an embodiment of the present invention comprises a step of feeding the discharged gas mixture to the second distillation column.

In step (7), the gas mixture may be fed to the middle or lower part of the second distillation column. Specifically, the gas mixture may preferably be fed to the middle position, or a lower position, of the second distillation column in order to perform more effective purification, but it is not limited thereto.

More specifically, the position at which the gas mixture is fed to the second distillation column in step (7) may be the same as, or lower than, the position at which the first bottom product is fed to the second distillation column in step (3). As the position at which the gas mixture in step (7) is fed is the same as, or lower than, the position at which the first bottom product is fed to the second distillation column in step (3), NMP can be purified more effectively. In particular, since high-purity NMP, as a final product, is discharged to the outside of the system through the top of the second distillation column, the first bottom product and the gas mixture must be fed to the middle position, or a lower position, of the second distillation column to increase the efficiency of NMP purification. As the feeding position of the gas mixture having a low content of NMP is set to be relatively lower than the feeding position of the first bottom product, more efficient purification can be carried out.

### NMP purification apparatus

The apparatus for purifying NMP according to another embodiment of the present invention comprises: a supply unit for supplying waste NMP; a first distillation column for performing first purification of the supplied waste NMP through distillation, discharging an overhead product containing water separated by the first purification to the outside of the system through its top, and discharging a first bottom product through its bottom; a second distillation column for performing second purification of the first bottom product through distillation, discharging purified NMP separated by the second purification to the outside of the system through its top, and discharging a second bottom product through its bottom; and a filtrate recovery device for performing thermal treatment of the second bottom product, discharging a third bottom product separated by the thermal treatment to the outside of the system through its bottom, and discharging a gas mixture containing NMP through its top.

Fig. 2 illustrates an example of an NMP purification apparatus according to another embodiment of the present invention. Specifically, Fig. 2 illustrates an NMP purification apparatus composed of a supply unit (SD), a first distillation column (D1), a second distillation column (D2), and a filtrate recovery device (R).

First, waste NMP is supplied to a first distillation column (D1) through a supply unit (SD). The supply may be carried out through a first pump (P1). In such an event, the waste NMP may be preheated through a preheater (B1).

The first distillation column (D1) performs first purification by distilling the supplied waste NMP. An overhead product containing water separated by the first purification is discharged to the outside of the system through the top of the first distillation column (D1) (T-1), and a first bottom product is discharged through the bottom of the first distillation column (D1) (Y-1). The discharged first bottom product may be transferred to a second distillation column (D2) through a second pump (P2) (X-1) and may be circulated back to the first distillation column (D1) (S-1). In such an event, the first bottom product may be preheated through a first reboiler (B2) before it is fed back to the first distillation column (D1).

The second distillation column (D2) performs second purification by distilling the fed first bottom product. Purified NMP separated by the second purification is discharged to the outside of the system through the top of the second distillation column (D2) (T-2), and a second bottom product is discharged through the bottom of the second distillation column (D2) (Y-2). The purified NMP discharged from the system may be stored in a storage tank. In addition, the discharged second bottom product may be transferred to a filtrate recovery device (R) through a third pump (P3) (X-2) and may be circulated back to the second distillation column (D2) (S-2). In such an event, the second bottom product may be preheated through a second reboiler (B3) before it is fed back to the second distillation column (D2).

The filtrate recovery device (R) performs thermal treatment of the fed second bottom product. The third bottom product separated by the thermal treatment is discharged to the outside of the system through the bottom of the filtrate recovery device (R) (Y-3). In addition, the gas mixture containing NMP separated by the thermal treatment may be discharged through the top of the filtrate recovery device (R) and circulated to the second distillation column (D2) (S-3).

### [Explanation of Reference Numerals]

D1: first distillation column
D2: second distillation column
P1: first pump
P2: second pump
P3: third pump
R: filtrate recovery device
SD: supply unit
B1: preheater
B2: first reboiler
B3: second reboiler
T-1, T-2, Y-1, Y-2, Y-3: discharge steps
S-1, S-2, S-3: circulation steps
X-1, X-2: feeding steps

## Claims

1. A process for purifying NMP, which comprises:
(1) distilling waste NMP in a first distillation column to perform first purification;
(2) discharging an overhead product containing water separated by the first purification to the outside of the system through the top of the first distillation column; and discharging a first bottom product through the bottom of the first distillation column;
(3) feeding the discharged first bottom product to a second distillation column to perform second purification;
(4) discharging purified NMP separated by the second purification to the outside of the system through the top of the second distillation column; and discharging a second bottom product through the bottom of the second distillation column;
(5) feeding the discharged second bottom product to a filtrate recovery device to perform thermal treatment thereof;
(6) discharging a third bottom product separated by the thermal treatment to the outside of the system through the bottom of the filtrate recovery device; and discharging a gas mixture containing NMP through the top of the filtrate recovery device; and
(7) feeding the discharged gas mixture to the second distillation column.

2. The process for purifying NMP according to claim 1, wherein the waste NMP contains NMP in an amount of 70% by weight or more.

3. The process for purifying NMP according to claim 1, wherein, in step (1), the waste NMP is fed to the middle or upper part of the first distillation column.

4. The process for purifying NMP according to claim 1, wherein the first bottom product contains NMP in an amount of 99.9% by weight or more.

5. The process for purifying NMP according to claim 1, wherein the content of water in the first bottom product is 0.01% or less.

6. The process for purifying NMP according to claim 1, wherein, in step (3), the first bottom product is fed to the middle or lower part of the second distillation column.

7. The process for purifying NMP according to claim 1, wherein the second bottom product contains NMP in an amount of 99% by weight or more.

8. The process for purifying NMP according to claim 1, wherein, in step (7), the gas mixture is fed to the middle or lower part of the second distillation column.

9. The process for purifying NMP according to claim 1, wherein the position at which the gas mixture is fed to the second distillation column in step (7) is the same as, or lower than, the position at which the first bottom product is fed to the second distillation column in step (3).

10. The process for purifying NMP according to claim 1, wherein the first purification in step (1) is performed at a temperature of 50°C to 150°C and a pressure of 100 Torr to 130 Torr, and the second purification in step (3) is performed at a temperature of 120°C to 150°C and a pressure of 50 Torr to 90 Torr.

11. The process for purifying NMP according to claim 1, wherein, in step (5), the thermal treatment is performed at a temperature of 110°C to 140°C and a pressure of 60 Torr to 100 Torr under stirring at 20 rpm to 80 rpm.

12. The process for purifying NMP according to claim 1, wherein the second bottom product contains a component having a boiling point higher than that of NMP in an amount of 1.0% by weight or less.

13. The process for purifying NMP according to claim 1, wherein the third bottom product contains a component having a boiling point higher than that of NMP in an amount of 1.5% by weight or less.

14. The process for purifying NMP according to claim 1, wherein the gas mixture contains NMP in an amount of 98.5% by weight or more.

15. The process for purifying NMP according to claim 1, wherein the purified NMP in step (4) has a purity of 99.99% or higher.

16. An apparatus for purifying NMP, which comprises:
a supply unit for supplying waste NMP;
a first distillation column for performing first purification of the supplied waste NMP through distillation, discharging an overhead product containing water separated by the first purification to the outside of the system through its top, and discharging a first bottom product through its bottom;
a second distillation column for performing second purification of the first bottom product through distillation, discharging purified NMP separated by the second purification to the outside of the system through its top, and discharging a second bottom product through its bottom; and
a filtrate recovery device for performing thermal treatment of the second bottom product, discharging a third bottom product separated by the thermal treatment to the outside of the system through its bottom, and discharging a gas mixture containing NMP through its top.
